(19) Europäisches Patentamt
European Patent Office
Office européen des brevets

(11) **EP 3 773 429 B1**

(12) **EUROPEAN PATENT SPECIFICATION**

(45) Date of publication and mention
of the grant of the patent:
**01.12.2021 Bulletin 2021/48**

(21) Application number: **19711089.3**

(22) Date of filing: **15.03.2019**

(51) Int Cl.:
*A61K 8/26* (2006.01)    *A61K 8/29* (2006.01)
*A61K 8/35* (2006.01)    *A61Q 13/00* (2006.01)
*A61Q 19/10* (2006.01)    *A61K 8/02* (2006.01)
*A61K 8/19* (2006.01)    *C11D 3/50* (2006.01)
*C11D 17/00* (2006.01)

(86) International application number:
**PCT/EP2019/056612**

(87) International publication number:
**WO 2019/192825 (10.10.2019 Gazette 2019/41)**

(54) **MICROCAPSULES FOR USE IN COSMETIC COMPOSITIONS**

MIKROKAPSELN ZUR VERWENDUNG IN KOSMETISCHEN ZUSAMMENSETZUNGEN

MICROCAPSULES À UTILISER DANS DES COMPOSITIONS COSMÉTIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.04.2018 PCT/CN2018/081762
24.05.2018 EP 18173952**

(43) Date of publication of application:
**17.02.2021 Bulletin 2021/07**

(73) Proprietors:
• **Unilever Global IP Limited
Wirral, CH62 AZD (GB)**
Designated Contracting States:
**CY DE GB IE IT MT**
• **Unilever IP Holdings B.V.
3013 AL Rotterdam (NL)**
Designated Contracting States:
**AL AT BE BG CH CZ DK EE ES FI FR GR HR HU
IS LI LT LU LV MC MK NL NO PL PT RO RS SE SI
SK SM TR**

(72) Inventors:
• **JIN, Huajin
Shanghai, Changning Disitrict 200335 (CN)**
• **PAN, Xiaoyun
Shanghai, Changning Disitrict 200335 (CN)**
• **WANG, Jinfang
Shanghai, Changning Disitrict 200335 (CN)**

(74) Representative: **Fijnvandraat, Arnoldus et al
Unilever N.V.
Unilever Patent Group
Bronland 14
6708 WH Wageningen (NL)**

(56) References cited:
**EP-A1- 1 512 664**    **EP-A1- 2 862 562**
**WO-A1-99/21532**    **WO-A1-2017/178978**
**US-A- 5 691 303**    **US-A- 6 113 682**
**US-A1- 2017 049 682**

• **DATABASE WPI Week 199507 Thomson
Scientific, London, GB; AN 1995-048642
XP002785965, -& JP H06 329411 A (SUMITOMO
CHEM CO LTD) 29 November 1994 (1994-11-29)**
• **DATABASE WPI Week 201463 Thomson
Scientific, London, GB; AN 2014-R41436
XP002785966, -& JP 2014 169235 A (OSAKA
ZOSENSHO KK) 18 September 2014 (2014-09-18)**

**Description**

**Field of the invention**

The present invention relates to non-spherical microcapsule. In particular, the present invention is related to a microcapsule comprising inorganic particle and benefit agent wherein the microcapsule has a sphericity of 0.15 to 0.74.

**Background of the invention**

Many home care and personal care products seek to deliver benefit agents to substrates such as textiles, hard surfaces, hair and skin. To achieve a long-lasting performance, encapsulation of the benefit agent in microcapsule has been proposed as a means, in particular for the perfume. When applied, the microcapsules may be deposited onto the substrates, for example onto clothes, and broken by action of pressure and/or rubbing when consumers get dressed. The perfume is released and brings superior sensory to the consumers. US 2017/0049682 A1 relates to improved fragrance delivery from soap bars.

However, there is still much room for improvement from many aspects. One aspect is to improve the efficiency of the deposition of encapsulated microcapsules. Deposition aid is one way to improve the deposition efficiency. The use of deposition aids is however not always desired.

In addition, the ingredients in the home care or person care products, may affect the deposition efficiency of the microcapsules when the microcapsules are incorporated into the products. Therefore, we have recognized that there is a need to develop microcapsules with improved deposition efficiency onto skin even when they are included into the products. We developed a microcapsule comprising inorganic particle and benefit agent, wherein the microcapsule has a sphericity of 0.15 to 0.74. It was surprisingly found that the deposition efficiency was significantly improved by such microcapsules even when they were included into skin cleansing composition.

**Summary of the invention**

In a first aspect, the present invention is directed to a microcapsule comprising inorganic particle and benefit agent, wherein the microcapsule has a sphericity of 0.15 to 0.74, wherein (a) said benefit agent is distributed throughout the particle, (b) said microcapsule is not core-shell microcapsule having a single core and (c) said benefit agent comprises fragrance, pro-fragrance, organic sunscreen, skin lightening agent, anti-aging agent or a mixture thereof.

In a second aspect, the present invention is directed to a home or personal care composition comprising microcapsules of the present invention.

All other aspects of the present invention will more readily become apparent upon considering the detailed description and examples which follow.

**Detailed description of the invention**

Except in the examples, or where otherwise explicitly indicated, all numbers in this description indicating amounts of material or conditions of reaction, physical properties of materials and/or use may optionally be understood as modified by the word "about". All amounts are by weight of the composition, unless otherwise specified.

It should be noted that in specifying any range of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

"Length", "width" and "thickness" refers to the length, width and thickness of particles or microcapsule in an unaggregated state. The term "length" refers to the average dimension of a particle or microcapsule typically along the longitudinal axis. The term "width" refers to the average dimension of the particle or microcapsule perpendicular to the length and typically perpendicular to the longitudinal axis. The terms "thickness" refer to the average dimension of the particle or microcapsule that is perpendicular to the length and width. The length, width and thickness may be measured for example by scanning electron microscopy (SEM) by averaging the values of at least ten particles.

"Sphericity" is the measure of how closely the shape of an object approaches that of a mathematically perfect sphere. The sphericity of a particle is the ratio of the surface area of a sphere (with the same volume as the given particle) to the surface area of the particle.

"Specific surface area" as used herein refers to specific surface area determined according to Brunauer-Emmett-Teller method. The value of the specific surface area was measured by meeting the requirements set out in ASTM standard D 3663-78.

"Particle size" as used herein refers to particle diameter in non-aggregated state unless otherwise stated. For polydisperse samples having particulate with diameter no greater than 1 $\mu$m, diameter means the z-average particle size measured, for example, using dynamic light scattering (see international standard ISO 13321) with an instrument such as a Zetasizer Nano™ (Malvern Instruments Ltd, UK). For polydisperse samples having particulate with diameter greater than 1 $\mu$m, diameter means the apparent volume median diameter (D50, also known as x50 or sometimes d(0.5)) of the particles measurable for example, by laser diffraction using a system (such as a Mastersizer™ 2000 available from Malvern Instruments Ltd) meeting the requirements set out in ISO 13320.

Preferably the microcapsule has platy shape. Preferably, the microcapsule has a sphericity of at least 0.185, more preferably at least 0.205, even more preferably at least 0.22 and still even more preferably at least 0.3. Preferably, the microcapsule has a sphericity of no greater than 0.72, more preferably no greater than 0.6 and even more preferably no greater than 0.5.

The microcapsule in accordance with this invention is not core-shell microcapsule having a single core. The benefit agent is distributed throughout the particle. Preferably, the microcapsule comprises one single inorganic particle.

The microcapsule preferably has an average particle size of 0.5 to 50 microns, more preferably 0.8 to 35 microns, even more preferably from 3 to 25 microns, still even more preferably from 4 to 17 microns. Preferably, the inorganic particle has platy shape. Preferably, the inorganic particle has a sphericity of at least 0.185, more preferably at least 0.205, even more preferably at least 0.22 and still even more preferably at least 0.3. Preferably, the inorganic particle has a sphericity of no greater than 0.72, more preferably no greater than 0.6 and even more preferably no greater than 0.5.

The inorganic particle preferably comprises bismuth oxychloride, aluminum oxide, barium sulphate, boron nitride, zirconium oxide, mica, clay, silicate, talc, or a combination thereof. More preferably, the particle comprises boron nitride, mica or a combination thereof. Even more preferably, the particle comprises boron nitride. Preferably, the boron nitride is hexagonal boron nitride. Hexagonal boron nitride used herein also includes quasi hexagonal boron nitride. Preferably, the outermost side of the inorganic particle is boron nitride.

Preferably, the inorganic particle is a platy particle having multilayers. The benefit agent is preferably distributed in the interlayer of the multilayers of the particles. Preferably the inorganic particle comprises platy boron nitride, platy mica, or a combination thereof. The inorganic particle preferably has a specific surface area of 0.5 to 25 m2/g, more preferably 1.1 to 13 m2/g, even more preferably 1.4 to 13 m2/g and still even more preferably from 5 to 12 m2/g.

The weight ratio of the particle to the benefit agent is preferably from 1:4 to 20:1, more preferably from 1:2 to 5:1 preferably 1:1 to 4:1.

Various benefit agents can be incorporated into the particles. The benefit agents comprise fragrance, pro-fragrance, organic sunscreen, skin lightening agent, anti-aging agent or a mixture thereof. More preferably the benefit agent is selected from fragrance, pro-fragrance, organic sunscreen, skin lightening agent or a mixture thereof. Even more preferably the benefit agent is fragrance, pro-fragrance or a mixture thereof. Most preferably the benefit agent is fragrance.

Typically, the fragrance comprises components having a boiling point of less than 300, more preferably 100-250 Celsius, measured at one atmosphere. It is also advantageous to comprises components which have a LogP of less than 3.0 (i.e. those which will be partitioned into water).

The pro-fragrance can, for example, be a food lipid. Food lipids typically contain structural units with pronounced hydrophobicity. The majority of lipids are derived from fatty acids. In these 'acyl' lipids the fatty acids are predominantly present as esters and include mono-, di-, triacyl glycerols, phospholipids, glycolipids, diol lipids, waxes, sterol esters and tocopherols.

The fragrance is typically present in an amount of from 10-85% by total weight of the particle, preferably from 15 to 75% by total weight of the particle. The fragrance suitably has a molecular weight of from 50 to 500 Dalton. Pro-fragrances can be of higher molecular weight, being typically 1-10k Dalton.

The microcapsule may be prepared in any suitable process. However, it is preferable that the process for preparing the microcapsule comprises the step of mixing of inorganic particle with benefit agent at 0 to 40 °C. Preferably, the step of mixing is conducted under agitation

The end-product compositions of the invention may be in any physical form but preferably an aqueous-based liquid. The microcapsule of the invention may be advantageously incorporated into personal care or home care compositions but preferably a personal care composition. The home care composition is preferably an aqueous laundry detergent or an aqueous fabric conditioner. The composition is preferably a skin cleansing composition containing a cleansing surfactant.

Typically, the composition comprises the microcapsules at levels of from 0.001% to 10%, more preferably from 0.005% to 7.55%, more preferably from 0.01 to 5%, and most preferably from 0.1% to 2% by weight of the total composition.

The composition preferably comprises a cleansing surfactant. More than one cleansing surfactant may be included in the composition. The cleaning surfactant may be chosen from soap, non-soap anionic, cationic, non-ionic, amphoteric surfactant and mixtures thereof. Many suitable surface-active compounds are available and are fully described in the literature, for example, in "Surface-Active Agents and Detergents", Volumes I and II, by Schwartz, Perry and Berch. The preferred surface-active compounds that can be used are soaps, non-soap anionic, non-ionic surfactant, amphoteric surfactant or a mixture thereof.

Suitable non-soap anionic surfactants include linear alkylbenzene sulphonate, primary and secondary alkyl sulphates, particularly $C_8$ to $C_{15}$ primary alkyl sulphates; alkyl ether sulphates; olefin sulphonates; alkyl xylene sulphonates; dialkyl sulphosuccinates; fatty acid ester sulphonates; or a mixture thereof. Sodium salts are generally preferred.

Most preferred non-soap anionic surfactant are linear alkylbenzene sulphonate, particularly linear alkylbenzene sulphonates having an alkyl chain length of from $C_8$ to $C_{15}$. It is preferred if the level of linear alkylbenzene sulphonate is from 0 wt% to 30 wt%, more preferably from 1 wt% to 25 wt%, most preferably from 2 wt% to 15 wt%, by weight of the total composition.

Nonionic surfactants that may be used include the primary and secondary alcohol ethoxylates, especially the $C_8$ to $C_{20}$ aliphatic alcohols ethoxylated with an average of from 1 to 20 moles of ethylene oxide per mole of alcohol, and more especially the $C_{10}$ to $C_{15}$ primary and secondary aliphatic alcohols ethoxylated with an average of from 1 to 10 moles of ethylene oxide per mole of alcohol. Non ethoxylated nonionic surfactants include alkylpolyglycosides, glycerol monoethers, and polyhydroxyamides (glucamide). It is preferred if the level of non-ionic surfactant is from 0 wt% to 30 wt%, preferably from 1 wt% to 25 wt%, most preferably from 2 wt% to 15 wt%, by weight of a fully formulated composition comprising the microcapsules of the invention.

Suitable amphoteric surfactants preferably are betaine surfactants. Examples of suitable amphoteric surfactants include, but are not limited to, alkyl betaines, alkylamido betaines, alkyl sulfobetaines, alkyl sultaines and alkylamido sultaines; preferably, those having 8 to about 18 carbons in the alkyl and acyl group. It is preferred that the amount of the amphoteric surfactant is 0 to 20 wt%, more preferably from 1 to 10 wt%, by weight of the composition.

It is also possible to include certain mono-alkyl cationic surfactants. Cationic surfactants that may be used include quaternary ammonium salts of the general formula $R^1R^2R^3R^4N^+ X^-$ wherein the R groups are long or short hydrocarbon chains, typically alkyl, hydroxyalkyl or ethoxylated alkyl groups, and X is a counter-ion (for example, compounds in which $R^1$ is a $C_8$-$C_{22}$ alkyl group, preferably a $C_8$-$C_{10}$ or $C_{12}$-$C_{14}$ alkyl group, $R^2$ is a methyl group, and $R^3$ and $R^4$, which may be the same or different, are methyl or hydroxyethyl groups); and cationic esters (for example, choline esters).

Water-soluble skin benefit agents may optionally be formulated into the compositions of the invention. A variety of water-soluble skin benefit agents can be used and the level can be from 0.1 to 50% but preferably from 1 to 30% by weight of the composition. These materials include, but are not limited to, polyhydroxy alcohols. Preferred water-soluble skin benefit agents are glycerin, sorbitol and polyethylene glycol.

Water-insoluble skin benefit agents may also be formulated into the compositions as conditioners and moisturizers. Examples include silicone oils; hydrocarbons such as liquid paraffins, petrolatum, microcrystalline wax, and mineral oil; and vegetable triglycerides such as sunflower seed and cottonseed oils.

Some compositions may include thickeners. These may be selected from cellulosics, natural gums and acrylic polymers but not limited by this thickening agent types. Among the cellulosics are sodium carboxymethyl cellulose, hydroxyethyl cellulose, hydroxypropyl methylcellulose and combinations thereof. Suitable gums include xanthan, pectin, karaya, agar, alginate gums and combinations thereof. Among the acrylic thickeners are homopolymers and copolymers of acrylic and methacrylic acids including carbomers such as Carbopol 1382, Carbopol 982, Ultrez, Aqua SF-1 and Aqua SF-2 available from the Lubrizol Corporation. Amounts of thickener may range from 0.01 to 3% by weight of the active polymer (outside of solvent or water) in the compositions.

Preservatives can desirably be incorporated into the compositions of this invention to protect against the growth of potentially harmful microorganisms. Suitable traditional preservatives for compositions of this invention are alkyl esters of para-hydroxybenzoic acid. Other preservatives which have more recently come into use include hydantoin derivatives, propionate salts, and a variety of quaternary ammonium compounds. Particularly preferred preservatives are phenoxyethanol, methyl paraben, propyl paraben, imidazolidinyl urea, sodium dehydroacetate and benzyl alcohol. The preservatives should be selected having regard for the use of the composition and possible incompatabilities between the preservatives and other ingredients. Preservatives are preferably employed in amounts ranging from 0.01% to 2% by weight of the composition.

A variety of other optional materials may be formulated into the compositions. These may include: antimicrobials such as 2-hydroxy-4,2',4'-trichlorodiphenylether (triclosan), 2,6-dimethyl-4-hydroxychlorobenzene, and 3,4,4'-trichlorocarbanilide; scrub and exfoliating particles such as polyethylene and silica or alumina; cooling agents such as menthol; skin calming agents such as aloe vera; and colorants.

In addition, the compositions of the invention may further include 0.5 to 10% by weight of sequestering agents, such as tetra sodium ethylenediaminetetraacetate (EDTA), EHDP or mixtures; opacifiers and pearlizers such as ethylene glycol distearate, titanium dioxide or Lytron 621 (Styrene/Acrylate copolymer); all of which are useful in enhancing the appearance or properties of the product.

Preferably the composition comprises water in an amount of at least 5% by weight of the composition, more preferably at least 25%, even more preferably 40 to 90% and still even more preferably at least 60 to 85% by weight of the composition.

The following examples are provided to facilitate an understanding of the invention. The examples are not intended to limit the scope of the claims.

**Examples**

Materials

Table 1

| Particle | Trade Name | Chemistry | Supplier |
|---|---|---|---|
| A | YT-BY-3-0.2 | Boron Nitride | Yao Tian* |
| B | YT-BY-3-0.5 | Boron Nitride | Yao Tian* |
| 1 | YT-BY-3-01 | Boron Nitride | Yao Tian* |
| 2 | CARESS® BN02 | Boron Nitride | Kobo |
| 3 | CARESS® BN06 | Boron Nitride | Kobo |
| 4 | CARESS® BN09 | Boron Nitride | Kobo |
| 5 | CARESS® BN12 | Boron Nitride | Kobo |
| 6 | CARESS® BN15 | Boron Nitride | Kobo |
| 7 | CARESS® BN18 | Boron Nitride | Kobo |
| 8 | CARESS® BN30 | Boron Nitride | Kobo |
| 9 | YT-BY-3-40 | Boron Nitride | Yao Tian* |
| 10 | RonaFlair® Low Luster Pigment | Mica/$BaSO_4$/$TiO_2$ | Merck |
| 11 | Mica S-25 | Mica | Kobo |
| C | RonaFlair® Extender W | Mica/$TiO_2$ | Merck |
| * Yao Tian: Shanghai Yao Tian Nano Material Co., Ltd. | | | |

Example 1

This example demonstrates the preparation of microcapsules.

The microcapsules are prepared by procedures as follows. 50 mg of Hostasol Yellow 3G (Clariant) was dissolved in 20 g of delta-damascene (No. 937856 ex Beijing Beida Zhengyuan science and technology company) to obtain fluorescer-containing fragrance. 0.05 mL of fluorescer-containing fragrance was added into 0.1 g of particles listed in Table 1 in a glass bottle. The particles were mixed together with the fragrance until the fragrance was fully adsorbed by the particles. Then, the microcapsules were obtained.

Optical microscopy (DM2500P, Leica, Germany) equipped with a fluorescence light was used to observe the morphology of the microcapsules. Fluorescence imaging demonstrated that the fluorescence agent was distributed evenly throughout the particle which indicates that the fragrance (damascone) is distributed evenly throughout the microcapsule.

The microcapsules were also observed by scanning electron microscope (Hitachi S-4800, Japan). The microcapsules were platy cuboid-like in shape which has same length and width. The length (L) and thicknesses (T) were obtained by averaging the values of at least ten particles. The particles are considered as a thin cuboid having two square faces for calculation of sphericity. The sphericities of the particles (microcapsules) were calculated by following the equation:

$$\text{Sphericity} = \left(\frac{\sqrt[3]{36\pi L^4 T^2}}{2L^2 + 4LT}\right)$$

Table 2 shows the lengths, thicknesses, and calculated sphericities of the microcapsules (particles).

Table 2

| Particle (microcapsule) | Length/$\mu$m | Thickness/$\mu$m | Sphericity |
|---|---|---|---|
| A | 0.2 | 0.2 | 0.81 |
| B | 0.5 | 0.3 | 0.78 |
| 1 | 1 | 0.3 | 0.68 |
| 2 | 2 | 0.5 | 0.64 |
| 3 | 6 | 0.5 | 0.40 |
| 4 | 9 | 0.5 | 0.32 |
| 5 | 12 | 0.5 | 0.27 |
| 6 | 15 | 0.5 | 0.23 |
| 7 | 18 | 0.5 | 0.21 |
| 8 | 30 | 0.8 | 0.20 |
| 9 | 40 | 0.8 | 0.17 |
| 10 | 15 | 0.3 | 0.17 |
| 11 | 25 | 0.5 | 0.17 |
| C | 20 | 0.3 | 0.14 |

Example 2

This example demonstrates the deposition efficiency of the microcapsules.

A typical skin wash procedure is shown as follows. A pig skin piece (2 cm $\times$ 2 cm) was placed in a Petri dish, to which 25 mg of formulation in Table 3 was added together with 3 mg of microcapsules as prepared in Example 1. The skin, with the mixture onto it, was then gently rubbed with another piece of skin for 30 seconds and the two pieces were rinsed with total 250 mL of tap water. The two pieces of skin were then immersed in a vial containing 10 mL of ethanol for one hour. 200 $\mu$L of the extraction liquid (ethanol) was withdrawn from the vial and added to a 96-well microplate for fluorescence measurement (excitation: 480 nm, emission: 520 nm) to afford a reading of $E_1$.

Table 3

| Ingredient | Weight % |
|---|---|
| Sodium Laureth Sulfate | 12.86 |
| Cocamidopropyl Betaine | 5.67 |
| Cocamide MEA | 1.35 |
| Acrylates Copolymer | 6.00 |
| Tetrasodium EDTA | 0.13 |
| Citric Acid Monohydrate | 0.10 |
| Sodium Hydroxide | 0.20 |
| Polypropylene glycol-400 | 0.70 |
| Sodium Chloride | 0.15 |
| Iodopropynyl Butylcarbamate | 0.07 |

(continued)

| Ingredient | Weight % |
|---|---|
| Phenoxyethanol | 0.60 |
| Water | to 100 |

Another two skin pieces were placed in a Petri dish, to which 3 mg of microcapsules was added. The skin pieces with the microcapsule were immediately immersed in a vial containing 10 mL of ethanol for one hour. 200 μL of the extraction liquid was withdrawn from the vial and added to a 96-well microplate for fluorescence measurement (excitation: 480 nm, emission 520: nm) to afford a reading of Eo.

In addition, two skin pieces were immersed in a vial containing 10 mL of ethanol for one hour. 200 μL of the extraction liquid was withdrawn from the vial and added to a 96-well microplate for fluorescence measurement (excitation: 480 nm, emission: 520 nm) to afford a reading of $E_b$.

The deposition efficiency was calculated according to the equation: Deposition efficiency = $(E_1-E_b) / (E_0-E_b) \times 100\%$.

The deposition efficiencies for the microcapsules were listed in Table 4.

Table 4

| Microcapsule | Sphericity | Deposition efficiency (%) |
|---|---|---|
| A | 0.81 | 4.1 ± 0.1 |
| B | 0.78 | 4.7 ± 0.7 |
| 1 | 0.68 | 10.1 ± 1.1 |
| 2 | 0.64 | 9.2 ± 0.8 |
| 3 | 0.40 | 17.4 ± 8.2 |
| 4 | 0.32 | 16.0 ± 1.2 |
| 5 | 0.27 | 14.7 ± 0.3 |
| 6 | 0.23 | 14.2 ± 5.3 |
| 7 | 0.21 | 11.3 ± 2.0 |
| 8 | 0.20 | 9.3 ± 2.3 |
| 9 | 0.17 | 6.3 ± 0.4 |
| 10 | 0.17 | 6.3 ± 0.3 |
| 11 | 0.17 | 6.7 ± 0.8 |
| C | 0.14 | 4.1 ± 0.3 |

Example 3

This example demonstrates the stability of the microcapsules in the formulation.

Raw material: Thymol was supplied by Sigma and Terpineol was supplied by Aldrich. Model fragrance (TT) was prepared by mixing Thymol and Terpinel together at the weight ratio of 2:5 to form homogeneous liquid.

Body wash formulation as in Table 3 above was prepared.

Preparation of "TT (Control)": 2mg TT was mixed with 25mg body wash formulation, and the mixture was allowed to stand at room temperature for 1 day before deposition test.

Preparation of "BN-TT": 4mg TT was mixed with 8mg CARESS® BN06, and the mixture was stirred for 1minute to make TT fully adsorbed by BN06. Then the mixture was allowed to stand at room temperature for 5 days in a sealed bottle to get the microcapsule BN-TT. Then, 6mg BN-TT was mixed with 25mg body wash formulation. The mixture was used for deposition test immediately (fresh) or was allowed to stand at room temperature for 1 day before deposition test (1-day aged).

Deposition test:

A typical skin wash procedure is shown as follows. A pig skin piece (2 cm × 2 cm) was placed in a Petri dish, to which the control or BN-TT sample that prepared according to above procedure was added. The skin, with the mixture onto it, was then gently rubbed with another piece of skin for 30 seconds and the two pieces were rinsed with total 250 mL of tap water. The two pieces of skin were then immersed in a vial containing 10 mL of methanol and treated with 10mins ultrasonic process. GC analysis was then conducted to determine the amount of TT (Terpineol as marker) in the methanol solution and the data was recorded as A1.

Another two skin pieces were placed in a Petri dish, to which 2mg of TT was added. The skin pieces with TT were immediately immersed in a vial containing 10 mL of methanol and treated with 10mins ultrasonic process. GC analysis was then conducted to determine the amount of TT (Terpineol as marker) in the methanol solution and the data was recorded as A0.

The deposition efficiency was calculated according to the equation: Deposition efficiency (%) = A1 / A0 × 100.

The deposition efficiencies for the microcapsules were listed in Table 5 below.

Table 5

| Reference No. | Sample | Deposition efficiency (100%) |
|---|---|---|
| D | TT (Control) | 12.5±1.2 |
| 12 | BN-TT capsule (fresh) | 23.5±4.6 |
| 13 | BN-TT capsule (1-day aged) | 23.4±0.4 |

The data in Table 5 above indicates that freshly prepared microcapsules (Reference No. 12) and 1-day aged microcapsules (Reference No.13) can deliver similar deposition efficacy, thereby indicates that the microcapsule as per the invention can be stable in the body wash formulation.

Example 4

This example demonstrates the difference between a microcapsule comprising inorganic particle with benefit agent and simple mixture of inorganic particle with benefit agent in the formulation.

Raw material:

Plate material: CARESS® BN15 (Boron Nitride with average size 15 micron) was supplied by KOBO;

Body Wash formulation details could be found in above table.

The ingredients of the multi component model fragrance was supplied by Sigma-Aldrich and the model fragrance was prepared according to below composition in Table 6.

Table 6

| Ingredient | CAS number | Concentration (%) |
|---|---|---|
| Hexyl Acetate | 142-92-7 | 1.2 |
| Limonene | 5989-27-5 | 1.8 |
| Citronellol | 106-22-9 | 21.5 |
| Lilial | 80-54-6 | 37 |
| beta pinene | 127-91-3 | 2.5 |
| benzyl acetate | 140-11-4 | 12 |
| Dihydromyrcenol | 18479-58-8 | 12 |
| PEA | 60-12-8 | 12 |

Experiment procedure:

Preparation of "Control": 24.88g body wash base and 0.13g fragrance were weighed into a sample cup and gently blended.

Preparation of sample "Fragrance@BN": 1.0g of BN 15 was weighed into a 20ml GC vial with hermetic screw cap lid with septa. A small stir bar was added to the vial. 0.5g of fragrance was added drop wise using a syringe with stirring to disperse the oil into the particle. The sample(s) were subsequently mixed by hand using a spatula to ensure all oil was dispersed into the BN15 particle. Active in particle was 33.3%. Then, 24.88g body wash base and 0.38g particle (33.3% active) were weighed into a sample cup and gently blended.

Preparation of sample "Fragrance+BN": 24.88g body wash base, 0.25g BN15 particle (without active) and 0.13g fragrance were weighed into a sample cup and gently blended.

GCMS headspace analysis: GCMS equilibrium headspace experiments were used to measure the amount of fragrance available in the headspace over the body wash samples. The assumption made here is that suppressed headspace over the sample, compared to a control with identical amount of fragrance loading, will give an indication of fragrance affinity for the particle vs the body wash, i.e. fragrance that encapsulated in the particle. All the Controls and Samples were weighed into 20ml GC vials, allowed to equilibrate for 24 hours and run using SPME sampling using an MS detector. The percentage of unencapsulated fragrance in samples was calculated according to below equation and shown in Table 7:

$$\text{Unencapsulated fragrance (\%)} = \frac{\text{Perfume intensity of Sample}}{\text{Perfume intensity of Control}} * 100$$

The Unencapsulated fragrance in Control was normalized as 100%.

Table 7

| Reference No. | E | F | 14 |
|---|---|---|---|
| Fragrance | Unencapsulated fragrance in Control (%) | Unencapsulated fragrance in Fragrance+BN (%) | Unencapsulated fragrance in Fragrance@BN (%) |
| Beta Pinene | 100 | 93.4 | 45.1 |
| Hexyl Acetate | 100 | 101.1 | 76.2 |
| Limonene | 100 | 94.8 | 28.4 |
| Citronellol | 100 | 97.0 | 41.4 |
| Lilial | 100 | 99.9 | 32.0 |

The data in Table 7 showed that the percentages of unencapsulated fragrance in "Fragrance+BN" samples were all above 90%, which indicated the low affinity between fragrance and particle if they were mixed with formulation separately. However, the percentages of unencapsulated fragrance in "Fragrance@BN" samples were lower than that of "Fragrance+BN" samples in most cases, which indicated the relatively high affinity between fragrance and particle in the microcapsule as per the invention (Reference No. 14).

**Claims**

1. A microcapsule comprising inorganic particle and benefit agent, wherein the microcapsule has a sphericity of 0.15 to 0.74, wherein: (a) said benefit agent is distributed throughout the particle, (b) said microcapsule is not core-shell microcapsule having a single core; and (c) said benefit agent comprises fragrance, pro-fragrance, organic sunscreen, skin lightening agent, anti-aging agent or a mixture thereof.

2. The microcapsule according to claim 1 wherein the particle comprises bismuth oxychloride, aluminum oxide, barium sulphate, boron nitride, zirconium oxide, mica, clay, silicate, talc, or a combination thereof, preferably, the particle comprises boron nitride, mica or a combination thereof.

3. The microcapsule according to claim 1 or 2 wherein the microcapsule has platy shape.

4. The microcapsule according to any one of the preceding claims wherein the microcapsule comprises one single inorganic particle.

5. The microcapsule according to any one of the preceding claims wherein the benefit agent is fragrance, pro-fragrance, or a mixture thereof, preferably, the benefit agent is fragrance.

6. The microcapsule according to any one of the preceding claims wherein the weight ratio of the particle to the benefit agent is from 1:4 to 20:1, preferably 1:1 to 4:1.

7. The microcapsule according to any one of the preceding claims wherein the microcapsules has a sphericity of from 0.18 to 0.74, preferably from 0.205 to 0.6, more preferably from 0.3 to 0.5.

8. The microcapsule according to any one of the preceding claims wherein the particle has a specific surface area of 0.5 to 25 $m^2$/g, preferably 1.1 to 12 $m^2$/g.

9. The microcapsule according to any one of the preceding claims wherein the particle has an average particle size of 0.5 to 50 microns, preferably from 3 to 25 microns, and more preferably from 4 to 17 microns.

10. A home or personal care composition comprising microcapsules of any one of the preceding claims, preferably the microcapsule is present in amount of 0.01 to 2% by weight of the composition.

11. The composition according to claim 10 wherein the composition comprises a cleansing surfactant.

12. The composition according to claim 10 or 11 wherein the composition is a skin cleansing composition.

**Patentansprüche**

1. Mikrokapsel, umfassend ein anorganisches Teilchen und ein Vorteilsmittel, wobei die Mikrokapsel eine Sphärizität von 0,15 bis 0,74 aufweist, wobei: (a) das Vorteilsmittel im ganzen Teilchen verteilt ist, (b) die Mikrokapsel keine Kern-Schale-Mikrokapsel mit einem einzelnen Kern ist; und (c) das Vorteilsmittel einen Duftstoff, eine Duftstoffvorstufe, ein Sonnenschutzmittel, einen Hautaufheller, ein Anti-Aging-Mittel oder eine Mischung davon umfasst.

2. Mikrokapsel nach Anspruch 1, wobei das Teilchen Wismutoxychlorid, Aluminiumoxid, Bariumsulfat, Bornitrid, Zirkoniumoxid, Glimmer, Ton, Silikat, Talk oder eine Kombination davon umfasst, wobei das Teilchen vorzugsweise Bornitrid, Glimmer oder eine Kombination davon umfasst.

3. Mikrokapsel nach Anspruch 1 oder 2, wobei die Mikrokapsel eine flache Form aufweist.

4. Mikrokapsel nach irgendeinem der vorhergehenden Ansprüche, wobei die Mikrokapsel ein einzelnes anorganisches Teilchen umfasst.

5. Mikrokapsel nach irgendeinem der vorhergehenden Ansprüche, wobei das Vorteilsmittel ein Duftstoff, eine Duftstoffvorstufe oder eine Mischung davon, vorzugsweise ein Duftstoff, ist.

6. Mikrokapsel nach irgendeinem der vorhergehenden Ansprüche, wobei das Gewichtsverhältnis des Teilchens zu dem Vorteilsmittel von 1:4 bis 20:1, vorzugsweise von 1:1 bis 4:1, beträgt.

7. Mikrokapsel nach irgendeinem der vorhergehenden Ansprüche, wobei die Mikrokapsel eine Sphärizität von 0,18 bis 0,74, vorzugsweise von 0,205 bis 0,6, bevorzugter von 0,3 bis 0,5, aufweist.

8. Mikrokapsel nach irgendeinem der vorhergehenden Ansprüche, wobei das Teilchen eine spezifische Oberfläche von 0,5 bis 25 $m^2$/g, vorzugsweise von 1,1 bis 12 $m^2$/g, aufweist.

**9.** Mikrokapsel nach irgendeinem der vorhergehenden Ansprüche, wobei das Teilchen eine durchschnittliche Teilchengröße von 0,5 bis 50 Mikrometer, vorzugsweise von 3 bis 25 Mikrometer, und bevorzugter von 4 bis 17 Mikrometer, aufweist.

**10.** Zusammensetzung zur häuslichen oder Körperpflege, umfassend Mikrokapseln nach irgendeinem der vorhergehenden Ansprüche, wobei die Mikrokapsel vorzugsweise in einer Menge von 0,01 bis 2 Gewichts-% der Zusammensetzung vorliegt.

**11.** Zusammensetzung nach Anspruch 10, wobei die Zusammensetzung ein reinigendes Tensid umfasst.

**12.** Zusammensetzung nach Anspruch 10 oder 11, wobei die Zusammensetzung eine Hautreinigungszusammensetzung ist.

**Revendications**

**1.** Microcapsule comprenant une particule inorganique et un agent bénéfique, dans laquelle la microcapsule présente une sphéricité de 0,15 à 0,74, dans laquelle : (a) ledit agent bénéfique est distribué d'un bout à l'autre de la particule, (b) ladite microcapsule n'est pas une microcapsule noyau-enveloppe présentant un noyau unique ; et (c) ledit agent bénéfique comprend un parfum, pro-parfum, écran solaire organique, agent éclaircissant la peau, agent anti-âge ou un mélange de ceux-ci.

**2.** Microcapsule selon la revendication 1, dans laquelle la particule comprend de l'oxychlorure de bismuth, oxyde d'aluminium, sulfate de baryum, nitrure de bore, oxyde de zirconium, mica, argile, silicate, stéatite, ou une combinaison de ceux-ci, de préférence, la particule comprend du nitrure de bore, mica ou une combinaison de ceux-ci.

**3.** Microcapsule selon la revendication 1 ou 2, dans laquelle la microcapsule présente une forme lamellaire.

**4.** Microcapsule selon l'une quelconque des revendications précédentes, dans laquelle la microcapsule comprend une seule particule inorganique.

**5.** Microcapsule selon l'une quelconque des revendications précédentes, dans laquelle l'agent bénéfique est un parfum, pro-parfum, ou un mélange de ceux-ci, de préférence, l'agent bénéfique est un parfum.

**6.** Microcapsule selon l'une quelconque des revendications précédentes, dans laquelle le rapport en masse de la particule à l'agent bénéfique est de 1 : 4 à 20 : 1, de préférence 1 : 1 à 4 : 1.

**7.** Microcapsule selon l'une quelconque des revendications précédentes, dans laquelle les microcapsules présentent une sphéricité de 0,18 à 0,74, de préférence de 0,205 à 0,6, encore mieux de 0,3 à 0,5.

**8.** Microcapsule selon l'une quelconque des revendications précédentes, dans laquelle la particule présente une surface spécifique de 0,5 à 25 $m^2$/g, de préférence 1,1 à 12 $m^2$/g.

**9.** Microcapsule selon l'une quelconque des revendications précédentes, dans laquelle la particule présente une taille moyenne de particule de 0,5 à 50 microns, de préférence de 3 à 25 microns, et encore mieux de 4 à 17 microns.

**10.** Composition pour le soin de la personne ou maison comprenant des microcapsules selon l'une quelconque des revendications précédentes, la microcapsule est de préférence présente dans une quantité de 0,01 à 2 % en masse de la composition.

**11.** Composition selon la revendication 10, dans laquelle la composition comprend un tensioactif nettoyant.

**12.** Composition selon la revendication 10 ou 11, dans laquelle la composition est une composition pour le nettoyage de la peau.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20170049682 A1